# EUROPÄISCHE PATENTSCHRIFT

(11) **EP 0 927 020 B1**
(45) Veröffentlichungstag und Bekanntmachung des Hinweises auf die Patenterteilung: **20.11.2002**
(21) Anmeldenummer: 97941963.7
(22) Anmeldetag: 25.08.1997
(51) Int. Cl.: A61K 7/06, A61K 7/09

(54) **VERFAHREN UND MITTEL ZUR DAUERHAFTEN VERFORMUNG VON KERATINFASERN**
PROCESS AND AGENTS FOR PERMANENTLY SHAPING KERATIN FIBRES
PROCEDE ET AGENTS POUR DONNER UNE FORME PERMANENTE A DES FIBRES KERATINIQUES

(30) Priorität: 02.09.1996 DE 19635481
(43) Veröffentlichungstag der Anmeldung: 07.07.1999
(73) Patentinhaber: Henkel Kommanditgesellschaft auf Aktien, 40191 Düsseldorf (DE)
(72) Erfinder: CORTEKAR, Hans-Wolfgang, D-42855 Remscheid (DE); OBERKOBUSCH, Doris, D-40591 Düsseldorf (DE)
(86) Internationale Anmeldenummer: EP9704622
(87) Internationale Veröffentlichungsnummer: WO98009606

(56) Entgegenhaltungen:
- EP-A- 0 256 462
- EP-A- 0 713 695
- WO-A-92/17155
- WO-A-96/09030
- GB-A- 2 153 865
- US-A- 3 910 289

## Beschreibung

Die Erfindung betrifft ein Verfahren zur dauerhaften Verformung von Keratinfasern, insbesondere von menschlichen Haaren, sowie zum Einsatz in diesem Verfahren geeignete Mittel.

Die dauerhafte Verformung von Keratinfasern wird üblicherweise so durchgefiihrt, daß man die Faser mechanisch verformt und die Verformung durch geeignete Hilfmittel festlegt. Vor und/oder nach dieser Verformung behandelt man die Faser mit der wäßrigen Zubereitung einer keratinreduzierenden Substanz (Wellmittel) und spült nach einer Einwirkungszeit mit Wasser oder einer wäßrigen Lösung. In einem zweiten Schritt behandelt man dann die Faser mit der wäßrigen Zubereitung eines Oxidationsmittels (Fixiermittel). Nach einer Einwirkungszeit wird auch dieses ausgespült und die Faser von den mechanischen Verformungshilfsmitteln (Wickler, Papilloten) befreit.

Das Wellmittel ist üblicherweise alkalisch eingestellt, damit die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Wenngleich dieses als Dauerwelle bezeichnete Verfahren heute in großem Umfang angewendet wird, kann es hinsichtlich mehrerer Parameter noch nicht als optimal angesehen werden.

Entscheidend für den Erreichen der gewünschten Umformung ist die sogenannte "Stärke" des Wellmittels, die durch die Menge und Art des Reduktionsmittels sowie die Alkalität des Wellmittels beeinflußt werden kann. Wellmittel, die eine zufriedenstellende Umformleistung garantieren, können jedoch bei strapaziertem, insbesondere bei oxidativ vorbehandeltem, Haar Schädigungen bis hin zum Haarbruch hervorrufen. Auch können in einzelnen Fällen Probleme im Kopfhautbereich auftreten.

Weiterhin reicht die übliche saure Einstellung des Fixiermittels in den meisten Fällen nicht aus, um dem Haar innerhalb der Einwirkzeit seinen ursprünglichen pH-Wert wiederzugeben. Dies führt dazu, daß mit einer Dauerwelle behandelte Haare in den ersten Tagen nach der Behandlung in hohem Maße insbesondere gegen mechanische Beanspruchung empfindlich sind.

Die EP-A-0 256 462 offenbart den Einsatz eines Nachbehandlungsmittels, das ein Aluminiumsalz in kombination mit Panthenol enthält.

Es wurde nun überraschenderweise gefunden, daß durch Einsatz einer speziellen Wirkstoffkombination insbesondere den beiden genannten Mißständen in unerwartet hohem Maße. abgeholfen werden kann. Durch Zugabe dieser

Wirkstoffkombination entweder zum Wellmittel selbst oder zu einem zusätzlichen Zwischenbehandlungsmittel vor der Fixierung gelingt es, die Wellkraft im Rahmen des Verfahrens so zu verstärken, daß es möglich ist, entweder die Alkalität des Wellmittels zu verringern, zu milderen Reduktionsmitteln überzugehen und/oder die Menge des Reduktionsmittels zu senken. Umgekehrt ist es möglich, durch Zusatz dieser Wirkstoffkombination zu üblichen Wellmittein bzw. Zwischenbehandlungsmitteln ein kräftigeres und länger haltbares Wellergebnis zu erhalten.

Gegenstand der Erfindung ist daher ein Verfahren zur dauerhaften Verformung von Keratinfasern, bei welchem
- man die Faser vor und/oder nach einer mechanischen Verformung mit einer wäßrigen Zubereitung einer keratinreduzierenden Substanz (Wellmittel) für eine Einwirkzeit behandelt,
- gewünschtenfalls das Wellmittel ausspült,
- gewünschtenfalls ein Zwischenbehandlungsmittel für eine Einwirkzeit aufträgt
- gewünschtenfalls das Zwischenbehandlungsmittel ausspült,
- die Faser dann mit einer wäßrigen Zubereitung eines Oxidationsmittels (Fixiermittel) innerhalb einer weiteren Einwirkungszeit fixiert und
- das Fixiermittel schließlich mit einer Spülung ausspült,
dadurch gekennzeichnet,
daß das Wellmittel und/oder das Zwischenbehandlungsmittel eine Wirkstoffkombination enthält, die besteht aus
- einem physiologisch verträglichen Salz (A), bestehend aus dem Anion einer anorganischen oder organischen Säure und einem zweiwertigen Kation oder Al³⁺,
und
- einem Vitamin oder Vitamin-Derivat (B), ausgewählt aus Vitamin C, Vitamin B₅ und deren Derivaten.

Die Einzelheiten der erfindungsgemäßen Lehre werden im folgenden anhand von Dauerwellmitteln geschildert. Die Lehre eignet sich aber in gleichem Maße und mit den gleichen Vorteilen zum Glätten von natürlich gekräuselten oder chemisch gewellten Haaren.

Erfindungsgemäß kann die genannte Wirkstofikombination im Wellmittel, im Zwischenbehandlungsmittel oder in beiden Mitteln enthalten sein.

In einer ersten bevorzugten Ausführungsform des erfindungsgemäßen Verfahrens ist diese Wirkstoffkombination nur im Wellmittel enthalten. In diesem Fall kann kann auf die Anwendung eines Zwischenbehandlungsmittels und somit auch dessen Ausspülen verzichtet werden. Die Einwirkzeit des Wellmittels auf das Haar beträgt dabei wie üblich ca. 15 - 40 Minuten.

In einer zweiten bevorzugten Ausführungsform des erfindungsgemäßen Verfahrens ist diese Wirkstoffkombination nur in einem Zwischenbehandlungsmittel enthalten. In diesem Falle beträgt die Einwirkzeit des Zwischenbehandlungsmittlels vorzugsweise 2 bis 15 Minuten, insbesondere 5 bis 10 Minuten. Danach kann das Zwischenbehandlungsmittel, beispielsweise mit klarem Wasser, ausgespült werden. Es ist jedoch im Rahmen dieser Ausführungsform des erfindungsgemäßen Verfahrens bevorzugt, das Zwischenbehandlungsmittel auf dem Haar zu belassen und direkt das Fixiermittel aufzutragen.

Ebenfalls Gegenstand der Erfindung sind Wellmittel und Zwischenbehandlungsmittel mit der erfindungsgemäßen Wirkstoffkombination.

Die folgenden Ausführungen zu der erfindungsgemäßen Wirkstoffkombination sind in gleichem Maße für deren Einsatz in Wellmitteln als auch in Zwischenbehandlungsmitteln gültig.

Die erste Komponente der erfindungsgemäßen Wirkstoffkombination ist ein physiologisch verträgliches Salz (A), bestehend aus dem Anion einer anorganischen oder organischen Säure und einem zweiwertigen Kation oder Al³⁺. Erfindungsgemäße Zwischenbehandlungsmittel enthalten zwingend ein physiologisch verträgliches Salz (A), bestehend aus dem Anion einer anorganischen oder organischen Säure und einem zweiwertigen Kation.

Bevorzugt wird das zweiwertige Kation eines Erdalkalimetalls oder Zink²⁺ eingesetzt. Besonders bevorzugt sind die zweiwertigen Kationen von Kalzium, Magnesium und Zink. Magnesiumsalze sind ganz besonders bevorzugt.

Die anorganische Säure, die das Anion der erfindungsgemäß eingesetzten Salze (A) bildet, wird bevorzugt ausgewählt aus Halogenwasserstoffsäuren, Schwefelsäure und Phosphorsäure. Besonders bevorzugt sind die Chloride der oben genannten Kationen.

Eine bevorzugte organische Säure, die das Anion der erfindungsgemäß eingesetzten Salze (A) bildet, ist Essigsäure.

Magnesiumchlorid und Magnesiumacetat stellen besonders bevorzugt eingesetzte Salze (A) im Rahmen der vorliegenden Erfindung dar.

Die zweite Komponente der erfindungsgemäßen Wirkstoffkombination ist ein Vitamin oder Vitamin-Derivat (B), ausgewählt aus Vitamin C, Vitamin B₅ und deren Derivaten.

Gemäß einer ersten bevorzugten Ausführungsform wird als Komponente (B) Ascorbinsäure eingesetzt. Bei der Wahl von Ascorbinsäure als Komponente (B) ist es weiterhin bevorzugt, dem erfmdungsgemäßen Mittel einen Stabilisator für Ascorbinsäure beizugeben. Stabilisatoren für Ascorbinsäure sind dem Fachmann bekannt und können einschlägigen Veröffentlichungen entnommen werden. Erfindungsgemäß bevorzugte Stabilisatoren sind Komplexbildner wie EDTA sowie Thioverbindungen.

Gemäß einer weiteren bevorzugten Ausführungsform wird Komponente (B) ausgewählt aus Pantothensäure, Panthenol, Panthenolestern und Panthenolethern. Erfindungsgemäß einsetzbare Panthonalester -und ether sind beispielsweise das Triacetat des Panthenols somit der Panthenolmonomethylester und dessen Monoacetat. Besonders bevorzugt im Rahmen dieser Ausführungsform ist die Verwendung des Panthenols.

Die Komponente (A) der erfindungsgemäßen Wirkstoffkombination ist bevorzugt in Mengen von 0,1 - 10 Gew.-%, bezogen auf das jeweilige Mittel, enthalten. Bevorzugt sind Mengen von 0,2 - 5, insbesondere 0,5 bis 3, Gew.-%.

Die Komponente (B) der erfindungsgemäßen Wirkstoffkombination ist bevorzugt in Mengen von 0,1 - 10 Gew.-%, bezogen auf das jeweilige Mittel, enthalten. Bevorzugt sind Mengen von 0.2 - 5, insbesondere 0,2 bis 2, Gew.-%.

In der erfindungsgemäßen Wirkstoffkombination sind die Wirkstoffe (A) und (B) bevorzugt in einem Mengenverhältnis von 10:1 bis 1:10 enthalten. Mengenverhältnisse von 1:2 bis 1:4 sind besonders bevorzugt.

Weiterhin enthalten die erfindungsgemäßen Mittel bevorzugt noch eine Di- oder Tricarbonsäure mit 2-6 Kohlenstoffatomen und 0-2 Hydroxygruppen. Beispiele für solche Säuren sind Oxalsäure, Malonsäure, Bernsteinsäure, Zitronensäure und Weinsäure. Unter diesen Säuren ist die Malonsäure besonders bevorzugt.

Soweit es sich bei den erfindungsgemäßen Mitteln um Wellmittel handelt, enthalten diese zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Alkalisalze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 Mol/kg bei einem pH-Wert von 5 bis 10, insbesondere von 7 bis 8,5, eingesetzt.

Weiterhin können die erfindungsgemäßen Wellmittel alle für Wellmittel bekannten Inhaltsstoffe enthalten, z.B.:
- anionische Tenside wie beispielsweise Seifen, Alkylsulfate und Alkylpolyglykolethersulfate, Salze von Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist, Acylsarcoside, Acyltauride, Acylisethionate, Sulfobernsteinsäuremono- und dialkylester, lineare Alkansulfonate, lineare Alpha-Olefinsulfonate, alpha-Sulfofettsäuremethylester und Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.
- zwitterionische Tenside wie beispielsweise Betaine und 2-Alkyl-3-carboxymethyl-3-hydroxyethyl-imidazoline.
- ampholytische Tenside, wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren.
- nichtionische Tenside wie beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl.
- kationische Tenside wie quartäre Ammoniumverbindungen mit 1-2 C₁₂₋₁₈-Alkylketten und 2 - 3 C₁₋₄-Alkylketten, Pyridinium- und Imidazoliniumsalze, Alkylamidoamine sowie die sogenannten Esterquats wie die unter dem Warenzeichen Stepantex^{R} vertriebenen Dialkylammoniummethosulfate und Methyl-hydroxyalkyl-dialkoyloxyalkyl-ammoniummethosulfate.
- Proteinhydrolysate wie beispielsweise Kollagen-Hydrolysate, Elastin-Hydrolysate, Keratin-Hydrolysate, Hydrolysate von Weizenproteinen, Milchproteinen, Eiweißproteinen, Seidenproteinen, Mandelproteinen, Sojaeiweiß sowie Proteinen aus Tierhäuten, Kollagenhydrolysat-Kondensate mit organischen Säuren, wie beispielsweise Ölsäure, Myristinsäure, Undecylensäure, Kokosfettsäure und Abietinsäure, und deren Salze, Elastinhydrolysat-Kondensate mit Fettsäuren und kationische Kollagenhydrolysate.
- Verdickungsmittel wie Agar-Agar, Guar-Gum, Alginate, Xanthan-Gum und Cellulose-Derivate wie Hydroxyethyl- und Methylhydroxypropyl-Cellulose,
- Strukturanten wie Glucose und Maleinsäure,
- Kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- Anionische, zwitterionische, amphotere und nichtionische Polymere wie beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvernetzte und mit Polyolen vernetzte Polyacrylsäuren, Acrylamidopropyl-trimethylammoniumchlorid/Acrylat-Copolymere, Octylacrylamid/-Methyl-methacrylat/tert.Butylammoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/ Vinylcaprolactam-Terpolymere sowie gegebenfalls derivatisierte Celluloseether und Silikone.
- Lösungsvermittler wie Ethanol, Isopropanol, Glycerin und Diethylenglykol,
- Substanzen zur Einstellung des pH-Wertes wie Natronlauge, Ammoniak, Ammoniumcarbonat, Ammoniumcarbamat und Citronensäure/Natriumcitrat-Puffer,
- Wirkstoffe wie Allantoin, Pyrrolidoncarbonsäuren und Pflanzenextrakte,
- Lichtschutzmittel,
- Komplexbildner wie NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Farbstoffe,
- Parfümöle, Dimethylisosorbid und Cyclodextrine,
- Perlglanzmittel wie Ethylenglykolmono- und -distearat sowie Fettsäurealkanolamide
- Treibmittel wie Propan-Butan-Gemische, N₂O, Dimethylether und Luft

Die Einwirkungszeit der Wellmittel auf dem Haar beträgt in der Regel 15 bis 40 Minuten, wobei die Beschaffenheit des Haares, die bereits durchgeführten chemischen Haarbehandlungen, der gewünschte Verformungsgrad, die Größe der verwendeten mechanischen Verformungshilfe (Haarwickler) und die Art des Keratinreduktionsmittels weitere Einflußgrößen sind.

Die erfindungsgemäßen Wellmittel können als gebrauchsfertige Mischungen formuliert werden, die vom Friseur oder Endverbraucher direkt angewendet werden können. Es hat sich in manchen Fällen aber als vorteilhaft oder notwendig erwiesen, wenn die Mittel als sogenannte 2-Komponenten-Mischungen formuliert werden, die erst vom Anwender zum gebrauchsfertigen Wellmittel vermischt werden. In diesem Fall enthält eine Formulierung das Reduktionsmittel in einem geeigneten Träger, z.B. Wasser oder einer Emulsion.

Die erfindungsgemäßen Zwischenbehandlungsmittel können die gleichen zusätzlichen Komponenten enthalten wie die erfindungsgemäßen Wellmittel. Üblicherweise werden sie aber frei von reduzierenden Mercaptanen sein oder diese nur in geringen Mengen, beispielsweise zur Stabilisierung der Ascorbinsäure, enthalten.

Die erfindungsgemäßen Verfahren unterliegen hinsichtlich der Verwendung der bekannten Fixiermittel keinerlei Beschränkungen.

Zwingender Bestandteil der Fixiermittel sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger H₂O₂-Zubereitungen, die üblicherweise etwa 0,5 bis 3,0 Gew.-% H₂O₂ enthalten, liegt bevorzugt bei 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 und der pH-Wert der Lösungen wird auf 4 bis 7 eingestellt. Gleichfalls geeignet sind Fixiermittel auf enzymatischer Basis (Peroxidasen), die keine oder nur geringe Mengen an Oxidationsmitteln, insbesondere H₂O₂, enthalten.

Die erfindungsgemäßen Fixiermittel können als Feststoffe formuliert werden. Sie enthalten das Oxidationsmittel dann in Form eines Festkörpers, z.B. Kalium- oder Natriumbromat. Erst kurz vor der Anwendung werden diese Mittel dann mit Wasser versetzt. Es kann ebenfalls bevorzugt sein, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile enthält, werden ebenfalls erst kurz vor der Anwendung vermischt.

Spülungen werden innerhalb des erfindungsgemäßen Verfahrens üblicherweise mit Wasser durchgeführt, dem anorganische Salze zugefügt werden können.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, CO₂, Luft, N₂O, Dimethylether, Fluorchlorkohlenwasserstofftreibmitteln oder Gemischen davon in Aerosolbehältern mit Schaumventil abgefüllt werden.

### Beispiele

### Allgemeine Vorschrift zur Bestimmung des Wellwertes nach Kirby

Zur Messung wurde eine 0,5 g schwere und 25 cm lange, glatte, an beiden Enden gleich dick abgebundene Haarsträhne mit dem Wellmittel befeuchtet. Diese wurde eng, aber ohne Spannung, um die Stifte des Wellbrettes gelegt, nochmals mit Wellmittel getränkt und 30 Min. bei 37 °C gewellt. Die Strähne wurde auf dem Brett gespült, 10 Min. in eine Fixierlösung von Zimmertemperatur getaucht, gespült, dann erst vorsichtig vom Wellbrett gelöst und 5 Min. in ein Wasserbad von 30 °C gelegt. Die lineare Entfernung der 1. und der 6. Welle (5 Wellen !) = Bₒ = Wellwert wurde gemessen. Weitere Einzelheiten zum Verfahren sind der Veröffentlichung von D.H. Kirby KPDR Heft 1/2, Seiten 3-6 (1958) zu entnehmen.

Es wurden folgende Mittel untersucht (alle Angaben sind Gew.-%):

| Komponente | Wellmittel Erfindung | Wellmittel Vergleich | Zwischenbehandlungsmittel Erfindung |
|---|---|---|---|
| Thioglykolsäure | 8,0 | 8,0 | - |
| Eumulgin® HRE 60¹ | 2,0 | 2,0 | - |
| Cremophor® RH 40² | 2,0 | 2,0 | 2,0 |
| EDTA | 0,2 | 0,2 | 0,1 |
| Harnstoff | 1,0 | 1,0 | - |
| Ammoniumcarbonat | 1,0 | 1,0 | - |
| Ascorbinsäure | 2,0 | - | 2,0 |
| MgCl₂ | 1,0 | - | 1,0 |
| Parfümöl | 0,5 | 0,5 | 0,2 |
| Ammoniak | ad pH 8 | ad pH 8 | - |
| Wasser | ad 100 | ad 100 | ad 100 |

| | | | |
|---|---|---|---|
| ¹ hydriertes Rizinusöl + 45 Ethylenoxid (CTFA-Bezeichnung: PEG-40-Hydrogenated-Castor-Oil) (BASF) | | | |
| ² hydriertes Rizinusöl + 60 Ethylenoxid (CTFA-Bezeichnung: PEG-60-Hydrogenated-Castor-Oil) (HENKEL) | | | |

| Komponente | Fixiermittel |
|---|---|
| Wasserstoffperoxid (50%) | 5,8 |
| EDTA | 0,2 |
| Dehyquart® E³ | 3,0 |
| Croquat® WKP⁴ | 0,5 |
| Parfümöl | 0,2 |
| Wasser | ad 100 |

| | |
|---|---|
| ³ N-(2-Hydroxyhexadecyl-1)-N,N-dimethyl-N-2-hydroxyethylammoniumchlorid (28 % Aktivsubstanz in Wasser; CTFA-Bezeichnung: Hydroxycetyl Hydroxyethyl Dimonium Chloride) (HENKEL) | |
| ⁴ kationisches Keratinhydrolysat (CTFA-Bezeichnung: Cocodimonium Hydroxypropyl Hydrolyzed Keratin) (CRODA) | |

Die bei den unterschiedlichen Verfahrensweisen erhaltenen Wellwerte nach Kirby sind in der folgenden Tabelle aufgeführt:

| angewendete Mittel | Wellwert [cm] |
|---|---|
| Wellmittel (Erfindung) | |
| Zwischenspülung | |
| Fixierung | |
| Spülung | 9,5 |
| | |
| Wellmittel (Vergleich) | |
| Zwischenspülung | |
| Fixierung | |
| Spülung | 10,0 |
| | |
| Wellmittel (Vergleich) | |
| Zwischenspülung | |
| Zwischenbehandlungsmittel (Erfindung) | |
| Fixierung | 9,5 |

## Patentansprüche

1. Verfahren zur dauerhaften Verformung von Keratinfasern, bei welchem
- man die Faser vor und/oder nach einer mechanischen Verformung mit einer wäßrigen Zubereitung einer keratinreduzierenden Substanz (Wellmittel) für eine Einwirkzeit behandelt,
- gewünschtenfalls das Wellmittel ausspült,
- gewünschtenfalls ein Zwischenbehandlungsmittel für eine Einwirkzeit auf trägt,
- gewünschtenfalls das Zwischenbehandlungsmittel ausspült,
- die Faser dann mit einer wäßrigen Zubereitung eines Oxidationsmittels (Fixiermittel) innerhalb einer weiteren Einwirkungszeit fixiert und
- das Fixiermittel schließlich mit einer Spülung ausspült,
**dadurch gekennzeichnet, daß** das Wellmittel und/oder das Zwischenbehandlungsmittel eine Wirkstoffkombination enthält, die besteht
aus
- einem physiologisch verträglichen Salz (A), bestehend aus dem Anion einer anorganischen oder organischen Säure und einem zweiwertigen Kation oder Al³⁺
und
- einem Vitamin oder Vitamin-Derivat (B), ausgewählt aus Vitamin C, Vitamin B₅ und deren Derivaten.

2. Verfahren nach Anspruch 1, **dadurch gekennzeichnet, daß** die Wirkstoffkombination im Wellmittel enthalten ist.

3. Verfahren nach Anspruch 1, **dadurch gekennzeichnet, daß** die Wirkstoffkombination im Zwischenbehandlungsmittel enthalten ist.

4. Mittel zur Verwendung als Wellmittel in einem Verfahren nach Anspruch 1 oder 2, **dadurch gekennzeichnet, daß** es eine Wirkstoffkombination enthält, die besteht aus
- einem physiologisch verträglichen Salz (A), bestehend aus dem Anion einer anorganischen oder organischen Säure und einem zweiwertigen Kation oder Al³⁺,
und
- einem Vitamin oder Vitamin-Derivat (B), ausgewählt aus Vitamin C, Vitamin B₅ und deren Derivaten.

5. Mittel zur Verwendung als Zwischenbehandlungsmittel in einem Verfahren nach Anspruch 1 oder 3, **dadurch gekennzeichnet, daß** es eine Wirkstoffkombination enthält, die besteht aus
- einem physiologisch verträglichen Salz (A), bestehend aus dem Anion einer anorganischen oder organischen Säure und einem zweiwertigen Kation,
und
- einem Vitamin oder Vitamin-Derivat (B), ausgewählt aus Vitamin C, Vitamin B₅ und deren Derivaten.

6. Mittel nach Anspruch 4 oder 5, **dadurch gekennzeichnet, daß** das zweiwertige Kation ausgewählt ist aus den Kationen von Erdalkalimetall und Zink.

7. Mittel nach Anspruch 6, **dadurch gekennzeichnet, daß** das zweiwertige Kation Magnesium ist.

8. Mittel nach einem der Ansprüche 4 bis 7, **dadurch gekennzeichnet, daß** die anorganische Säure ausgewählt ist aus Halogenwasserstoffsäuren, Schwefelsäure und Phosphorsäure.

9. Mittel nach Anspruch 8, **dadurch gekennzeichnet, daß** die anorganische Säure Chlorwasserstoffsäure ist.

10. Mittel nach einem der Ansprüche 4 bis 7, **dadurch gekennzeichnet, daß** die organische Säure Essigsäure ist.

11. Mittel nach einem der Ansprüche 4 bis 10, **dadurch gekennzeichnet**, das das Vitamin Ascorbinsäure ist.

12. Mittel nach einem der Ansprüche 4 bis 10, **dadurch gekennzeichnet, daß** das Vitamin oder Vitamin-Derivat ausgewählt ist aus Pantothensäure, Panthenol, Panthenolestern und Panthenolethern.

13. Mittel nach einem der Ansprüche 4 bis 12, **dadurch gekennzeichnet, daß** die Wirkstoffe (A) und (B) in einem Mengenverhältnis von 1:10 bis 10:1 enthalten sind.

14. Mittel nach einem der Ansprüche 4 bis 13, **dadurch gekennzeichnet, daß** es weiterhin eine Di- oder Tricarbonsäure mit 2-6 Kohlenstoffatomen und 0-2 Hydroxygruppen enthält.

15. Mittel nach Anspruch 14, **dadurch gekennzeichnet, daß** die Di- oder Tricarbonsäure ausgewählt ist aus Oxalsäure, Malonsäure, Bernsteinsäure, Zitronensäure und Weinsäure.

## Claims

1. A process for the permanent deforming of keratin fibers, in which
- before and/or after mechanical deforming, the fibers are treated for a certain time with an aqueous preparation of a keratin-reducing substance (wave lotion),
- the wave lotion is optionally rinsed out,
- an intermediate treatment is optionally applied for a certain time,
- the intermediate treatment, is optionally rinsed out,
- the fibers are then fixed for a certain time with an aqueous preparation of an oxidizing agent (fixing lotion) and
- finally the fixing lotion is rinsed out with a rinse,
**characterized in that** the wave lotion and/or the intermediate treatment contains a combination of active substances consisting of
- a physiologically compatible salt (A) consisting of the anion of an inorganic or organic acid and a divalent cation or Al³⁺
and
- a vitamin or vitamin derivative (B) selected from vitamin C, vitamin B₅ and derivatives thereof.

2. A process as claimed in claim 1, **characterized in that** the active-substance combination is present in the wave lotion.

3. A process as claimed in claim 1, **characterized in that** the active-substance combination is present in the intermediate treatment.

4. A formulation for use as a wave lotion in the process claimed in claim 1 or 2, **characterized in that** it contains an active-substance combination consisting of
- a physiologically compatible salt (A) consisting of the anion of an inorganic or organic acid and a divalent cation or Al³⁺
and
- a vitamin or vitamin derivative (B) selected from vitamin C, vitamin B₅ and derivatives thereof.

5. A formulation for use as an intermediate treatment in the process claimed in claim 1 or 3, **characterized in that** it contains an active-substance combination consisting of
- a physiologically compatible salt (A) consisting of the anion of an inorganic or organic acid and a divalent cation
and
- a vitamin or vitamin derivative (B) selected from vitamin C, vitamin B₅ and derivatives thereof.

6. A formulation as claimed in claim 4 or 5, **characterized in that** the divalent cation is selected from the cations of alkaline earth metals and zinc.

7. A formulation as claimed in claim 6, **characterized in that** the divalent cation is magnesium.

8. A formulation as claimed in any of claims 4 to 7, **characterized in that** the inorganic acid is selected from hydrohalic acids, sulfuric acid and phosphoric acid.

9. A formulation as claimed in claim 8, **characterized in that** the inorganic acid is hydrochloric acid.

10. A formulation as claimed in any of claims 4 to 7, **characterized in that** the organic acid is acetic acid.

11. A formulation as claimed in any of claims 4 to 10, **characterized in that** the vitamin is ascorbic acid.

12. A formulation as claimed in any of claims 4 to 10, **characterized in that** the vitamin or vitamin derivative is selected from pantothenic acid, panthenol, panthenol esters and panthenol ethers.

13. A formulation as claimed in any of claims 4 to 12, **characterized in that** components (A) and (B) are present in a quantity ratio of 1:10 to 10:1.

14. A formulation as claimed in any of claims 4 to 13, **characterized in that** it additionally contains a di- or tricarboxylic add containing 2 to 6 carbon atoms and 0 to 2 hydroxy groups.

15. A formulation as claimed in claim 14, **characterized in that** the di- or tricarboxylic acid is selected from oxalic acid, malonic acid, succinic acid, citric acid and tartaric acid.

## Revendications

1. Procédé de mise en forme permanente de fibres de kératine dans lequel
- on traite les fibres avant et/ou après une mise en forme mécanique avec une préparation aqueuse de substance qui réduit la kératine (agent d'ondulation) pendant un temps d'action,
- si désiré on élimine par rinçage l'agent d'ondulation,
- si désiré on applique un agent de traitement intermédiaire pendant un temps d'action,
- si désiré on élimine par rinçage l'agent de traitement intermédiaire,
- on fixe les fibres ensuite avec une préparation aqueuse d'un agent oxydant (agent de fixation) en l'espace d'un temps d'action supplémentaire, et
- on élimine par rinçage l'agent de fixation,
**caractérisé en ce que**
l'agent d'ondulation et/ou l'agent de traitement intermédiaire contient une combinaison de principes actifs qui consiste en
- un sel physiologiquement compatible (A) formé d'un anion d'un acide minéral ou organique et d'un cation divalent ou d'Al³⁺, et
- une vitamine ou un dérivé de vitamine (B) choisi parmi la vitamine C, la vitamine B5 et leurs dérivés.

2. Procédé selon la revendication 1,
**caractérisé en ce que**
la combinaison de principes actifs est contenue dans l'agent d'ondulation.

3. Procédé selon la revendication 1,
**caractérisé en ce que**
la combinaison de principes actifs est contenue dans l'agent de traitement intermédiaire.

4. Agent pour l'utilisation comme agent d'ondulation dans un procédé selon la revendication 1 ou la revendication 2,
**caractérisé en ce qu'**
il contient une combinaison de principes actifs qui consiste en
- un sel physiologiquement compatible (A) formé d'un anion d'un acide minéral ou organique et d'un cation divalent ou d'Al³⁺, et
- une vitamine ou un dérivé de vitamine (B) choisi parmi la vitamine C, la vitamine B5 et leurs dérivés.

5. Agent pour l'utilisation comme agent de traitement intermédiaire dans un procédé selon l'une quelconque des revendications 1 ou 3,
**caractérisé en ce qu'**
il convient une combinaison de principes actifs qui consiste en :
- un sel physiologiquement compatible (A) formé d'un anion d'un acide minéral ou organique et d'un cation divalent, et
- une vitamine ou un dérivé de vitamine (B) choisi parmi la vitamine C, la vitamine B5 et leurs dérivés.

6. Agent selon l'une quelconque des revendications 4 ou 5,
**caractérisé en ce que**
le cation divalent est choisi parmi les cations de métal alcalino-terreux et du zinc.

7. Agent selon la revendication 6,
**caractérisé en ce que**
le cation divalent est le magnésium.

8. Agent selon l'une quelconque des revendications 4 à 7,
**caractérisé en ce que**
l'acide minéral est choisi parmi les acides halohydriques, l'acide sulfurique et l'acide phosphorique.

9. Agent selon la revendication 8,
**caractérisé en ce que**
l'acide minéral est l'acide chlorhydrique.

10. Agent selon l'une quelconque des revendications 4 à 7,
**caractérisé en ce que**
l'acide organique est l'acide acétique.

11. Agent selon l'une quelconque des revendications 4 à 10,
**caractérisé en ce que**
la vitamine est l'acide ascorbique.

12. Agent selon l'une quelconque des revendications 4 à 10,
**caractérisé en ce que**
la vitarnine ou le dérivé de vitamine est choisi parmi l'acide pantothénique, le panthénol, les esters de panthénol et les éthers de panthénol.

13. Agent selon l'une quelconque des revendications 4 à 12,
**caractérisé en ce que**
les principes actifs (A) et (B) sont contenus dans un rapport en quantités allant de 1 :10 à 10 :1.

14. Agent selon l'une quelconque des revendications 4 à 13,
**caractérisé en ce qu'**
il contient en outre un acide dicarboxylique ou tricarboxylique ayant de 2 à 6 atomes de carbone et de 0 à 2 groupes hydroxy.

15. Agent selon la revendication 14,
**caractérisé en ce que**
l'acide dicarboxylique ou tricarboxylique est choisi parmi l'acide oxalique, l'acide malonique, l'acide succinique, l'acide critique et l'acide tartrique.
